(19) Europäisches Patentamt
European Patent Office
Office européen des brevets

(11) EP 3 061 430 A1

(12) EUROPEAN PATENT APPLICATION

(43) Date of publication:
31.08.2016 Bulletin 2016/35

(51) Int Cl.:
A61F 9/008 (2006.01)

(21) Application number: 16157534.5

(22) Date of filing: 26.02.2016

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 26.02.2015 JP 2015037415

(71) Applicant: Nidek co., Ltd.
Gamagori
Aichi (JP)

(72) Inventors:
• IWATA, Shinya
Gamagori,, Aichi (JP)
• HANEBUCHI, Masaaki
Gamagori,, Aichi (JP)
• MURAKAMI, Naho
Gamagori,, Aichi (JP)

(74) Representative: Hoefer & Partner Patentanwälte
mbB
Pilgersheimer Straße 20
81543 München (DE)

(54) **LASER EYE SURGERY APPARATUS AND ABERRATION COMPENSATION METHOD**

(57) A laser eye surgery apparatus includes: a laser light source configured to emit pulsed laser light; a scanner configured to scan the pulsed laser light emitted from the laser light source; a condenser configured to concentrate the pulsed laser light scanned by the scanner in a tissue of a patient's eye; and a plane-parallel plate which is provided in a region in which the pulsed laser light is converged or diverged on an optical path of the pulsed laser light from the laser light source to the patient's eye. The plane-parallel plate has a light incident plane to which the pulsed laser light is incident, and a light-emitting plane emitting the pulsed laser light incident from the light incident plane, and the light incident plane and the light-emitting plane are parallel to each other, or are the same plane.

FIG.1

**Description**

CROSS REFERENCE TO RELATED APPLICATION

This application is based upon and claims the benefit of priority of Japanese Patent Application No. 2015-037415 filed on February 26, 2016, the contents of which are incorporated herein by reference in its entirety.

BACKGROUND

The present disclosure relates to a laser eye surgery apparatus in which an operator treats a patient's eye by concentrating pulsed laser light in the tissue of the patient's eye, and an aberration compensation method for the laser eye surgery apparatus.

In the related art, a laser eye surgery apparatus, by which an operator treats (for example, cuts or fragments) a tissue by concentrating pulsed laser light on multiple target positions in the patient's eye, has been known. For example, a device disclosed in JP-T-2014-503259 forms a slit-shaped incision extending from the rear surface to the front surface of the cornea by scanning pulsed laser light. A surgical instrument is inserted into the eye through the formed corneal incision.

SUMMARY

In the laser eye surgery apparatus, an aberration associated with pulsed laser light occurs on various occasions. An astigmatism or a comma aberration may occur due to the scanning of pulsed laser light outside an optical axis, manufacturing errors of the apparatus, or the like. The occurrence of an aberration may cause a decrease in the quality of surgery.

A typical object of the present disclosure is to provide a laser eye surgery apparatus and an aberration compensation method by which an impact of an aberration can be appropriately suppressed.

According to a typical aspect (hereinafter, may also be referred to as "the aspect") of the present disclosure, there is provided a laser eye surgery apparatus in which an operator treats a patient's eye by concentrating pulsed laser light in a tissue of the patient's eye, the apparatus including: a laser light source configured to emit pulsed laser light; a scanner configured to scan the pulsed laser light emitted from the laser light source; and a plane-parallel plate which is provided in a region in which the pulsed laser light is converged or diverged on an optical path of the pulsed laser light from the laser light source to the patient's eye, and in which a light incident plane to which the pulsed laser light is incident, and a light-emitting plane emitting the pulsed laser light incident from the light incident plane are parallel to each other, or are the same plane.

According to another typical aspect of the present disclosure, there is provided an aberration compensation method by which an aberration of a laser eye surgery apparatus, in which an operator treats a patient's eye by concentrating pulsed laser light in a tissue of the patient's eye, is compensated for. The laser eye surgery apparatus includes: a laser light source configured to emit pulsed laser light; a scanner configured to scan the pulsed laser light emitted from the laser light source; and a plane-parallel plate which is provided in a region in which the pulsed laser light is converged or diverged on an optical path of the pulsed laser light from the laser light source to the patient's eye, and in which a light incident plane to which the pulsed laser light is incident, and a light-emitting plane emitting the pulsed laser light incident from the light incident plane are parallel to each other, or are the same plane. The method includes: an aberration acquisition step of acquiring an aberration which occurs in an optical system of the laser eye surgery apparatus due to manufacturing errors or usage degradation; and an angle adjustment step of adjusting the angle of the plane-parallel plate with respect to an optical axis of the pulsed laser light, according to the aberration acquired in the aberration acquisition step.

According to the laser eye surgery apparatus and the aberration compensation method, an impact of an aberration is appropriately suppressed.

Specifically, when the plane-parallel plate is provided in a region in which pulsed laser light is converged or diverged, an aberration may occur according to the angle between the optical axis of the pulsed laser light and the plane-parallel plate. Accordingly, the laser eye surgery apparatus in the embodiment is capable of appropriately suppressing an impact of an aberration by generating an aberration (that is, compensating for an undesirable aberration) via the plane-parallel plate so as to cancel out and reduce the undesirable aberration.

It is also considered that another method (for example, a method in which a lens provided on an optical path is moved in an optical axis direction) is used to compensate for an aberration. However, when the lens is moved in the optical axis direction, not only an aberration may be changed but also the concentration position of pulsed laser light may be changed in a Z-axis direction. In contrast, while suppressing a change in the concentration position in the Z-axis direction, the laser eye surgery apparatus in the embodiment is capable of suppressing an impact of an aberration by using the plane-parallel plate. In some cases, it is also possible to compensate for only at least one of an astigmatism and a comma aberration among various aberrations by using the plane-parallel plate.

The laser eye surgery apparatus may include a holding mechanism holding the plane-parallel plate. The angle (that is, the direction of the angle, and the amount of tilt angle) of the plane-parallel plate, which is held by the holding mechanism, with respect to the optical axis of pulsed laser light can be changed. In this case, an impact of an aberration is appropriately suppressed by adjusting the angle of the plane-parallel plate according to an occurring aberration.

The laser eye surgery apparatus may include a drive unit driving the holding mechanism of the plane-parallel plate. The laser eye surgery apparatus may include a controller controlling the drive unit. In this case, the laser eye surgery apparatus is capable of easily changing the angle of the plane-parallel plate according to whether or not compensation of an aberration is required. In a case where an aberration is changed during an operation, the controller also can appropriately change the angle of the plane-parallel plate according to the changing aberration during the operation.

An aberration may be changed according to the position (that is, at least one of an image height and the depth of a spot) of a spot, or the shape of the patient's eye. The laser eye surgery apparatus may adjust the amount of tilt angle of the plane-parallel plate with respect to the optical axis, according to at least one of the position of the spot and the shape of the patient's eye. Accordingly, the laser eye surgery apparatus is capable of appropriately adjusting the angle of the plane-parallel plate according to the occurring aberration.

When the direction of a normal line (extending in an X-Y plane) of the plane-parallel plate is changed, the mode of an aberration is also changed. The laser eye surgery apparatus may change the direction of the normal line (extending in the X-Y plane) of the plane-parallel plate according to the direction of a spot in the X-Y plane with respect to the optical axis. In this case, the laser eye surgery apparatus is capable of more appropriately compensating for an aberration by generating an aberration according to the position of the spot in the X-Y plane via the plane-parallel plate.

The laser eye surgery apparatus may drive the plane-parallel plate according to the state of an eyeball interface connected to the patient's eye. In this case, the laser eye surgery apparatus is capable of more appropriately suppressing an impact of an aberration even in a case where an aberration is changed by the eyeball interface.

The laser eye surgery apparatus may include two plane-parallel plates. For example, the laser eye surgery apparatus may tilt a first plane-parallel plate and a second plane-parallel plate at the same amount of tilt amount from an initial angle in opposite directions. In this case, an astigmatism is appropriately compensated for while a comma aberration is not changed. As a result, even if the angle of each of the two plane-parallel plates is changed, pulsed laser light passing through the plane-parallel plates passes through the same optical path as when the angle of each of the two plane-parallel plates is not changed and is set to the initial angle (that is, the pulsed laser light is offset from the optical path). The laser eye surgery apparatus may compensate for both of an astigmatism and a comma aberration by changing the respective angles of the two plane-parallel plates from the initial angle to different angles (for example, by tilting only one plane-parallel plate). The laser eye surgery apparatus may compensate for a diagonal astigmatism by tilting the two plane-parallel plates in directions which are not opposite to each other.

The plane-parallel plates may be disposed closer to the laser light source than (disposed on the upstream side of) an objective lens on the optical path of pulsed laser light. In this case, the distance between the objective lens and the patient's eye is easily reduced. Accordingly, an operator can perform a laser operation using a high numerical aperture via the laser eye surgery apparatus even if an excessively large objective lens is not used. In this case, the size of each of the plane-parallel plates can also be easily reduced.

BRIEF DESCRIPTION OF THE DRAWINGS

 Fig. 1 is a view illustrating the entire configuration of a laser eye surgery apparatus 1.
 Fig. 2 is a view illustrating the configuration of an aberration compensating device 5.
 Fig. 3 is a view illustrating the configuration of a holding mechanism 80 of a first plane-parallel plate 60.
 Fig. 4 is a flowchart illustrating a typical aberration compensation method.
 Fig. 5 is a flowchart illustrating a changing aberration compensation process executed by a CPU 47.
 Fig. 6 shows views illustrating a relationship between the direction of a spot with respect to an optical axis L and the directions of the first plane-parallel plate 60 and a second plane-parallel plate.

DESCRIPTION OF ILLUSTRATIVE EMBODIMENTS

Hereinafter, a typical embodiment of the present disclosure will be described. In the embodiment, a laser eye surgery apparatus 1, by which an operator can treat the cornea and the crystalline lens of a patient's eye E is exemplarily described. The term "treatment" implies the cutting, the fragmenting, or the like of the tissue of the patient's eye E.

<Entire Configuration>

Hereinafter, all configuration elements of the laser eye surgery apparatus 1 in the embodiment will be described sequentially from a laser light source 2 (that is, an upstream side on the optical path of pulsed laser light) to the patient's eye E (that is, a downstream side on the optical path of pulsed laser light).

The laser light source 2 emits pulsed laser light. In the embodiment, when the pulsed laser light emitted by the laser light source 2 is concentrated in the tissue of the patient's eye E, plasma occurs at a concentration position (spot), thereby cutting or fragmenting the tissue. Such a phenomenon may also be referred to as photodisruption. A device emitting pulsed laser light with a pulse width in the order of femtoseconds to picoseconds can be used as the laser light source 2. In the following description, a Z-axis direction refers to a direction along the optical axis of pulsed laser light emitted by the laser light source 2. An X-axis direction refers to one of directions perpendicular to the Z-axis direction. A Y-axis direction refers to a direction perpendicular to both the Z-axis direction and the X-axis direction.

An aiming light source 3 emits aiming light indicating the position of irradiated pulsed laser light. In the embodiment, a light source emitting visible laser light is used as the aiming light source 3. The aiming light source 3 may be omitted.

A dichroic mirror 4 is provided on the optical path (hereinafter, may also be simply referred to as an "optical path") of pulsed laser light. The dichroic mirror 4 multiplexes laser light emitted from the laser light source 2 and aiming light emitted from the aiming light source 3.

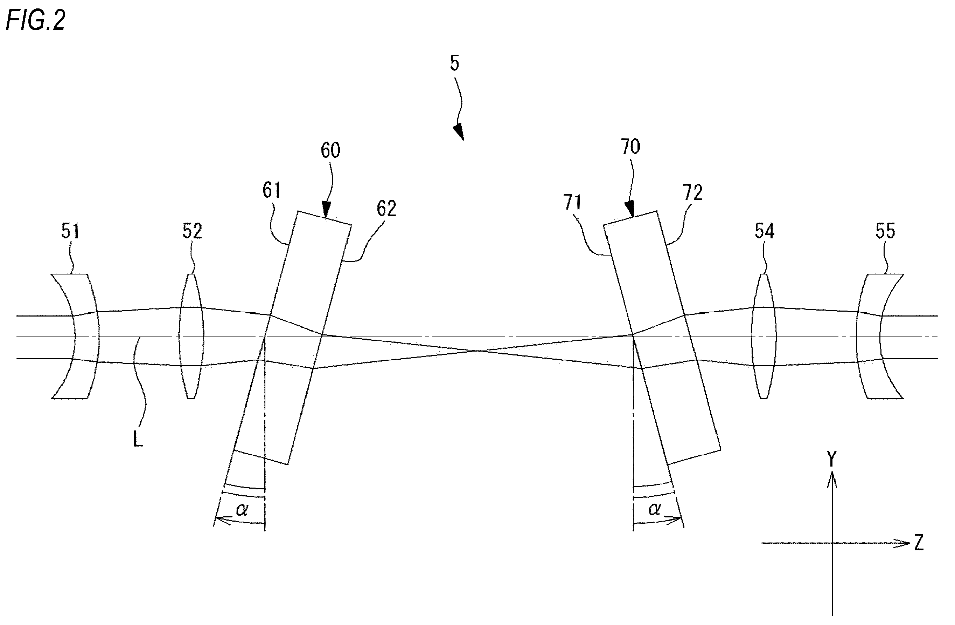

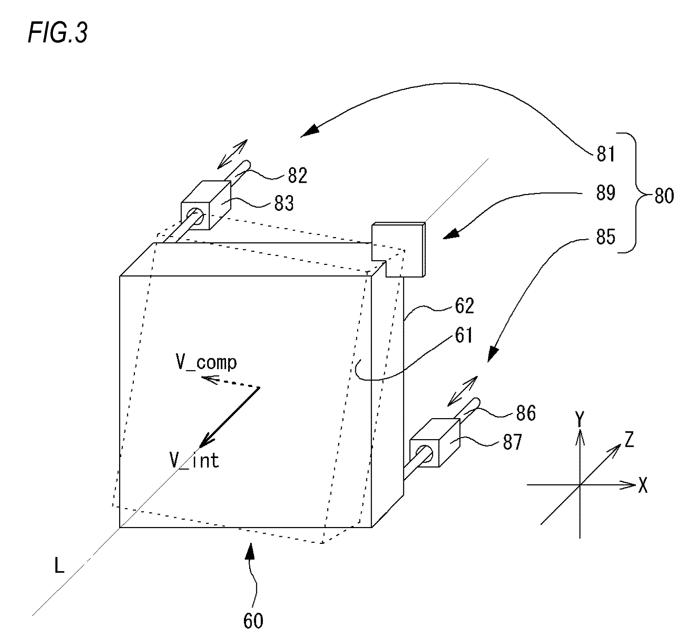

An aberration compensating device 5 suppresses an impact (for example, a decrease in the quality of surgery) of an aberration by compensating for the aberration occurring in the optical system of the laser eye surgery apparatus 1. That is, the aberration compensating device 5 generates an aberration which cancels out and reduces the aberration occurring in the optical system of the laser eye surgery apparatus 1. The aberration compensating device 5 will be described in detail later with reference to Figs. 2 and 3.

A zoom expander 13 is provided between the laser light source 2 and an X-Y scanner 25 (to be described later) on the optical path (specifically, in the embodiment, between the aberration compensating device 5 and a high-speed Z scanner 15 (to be described later)). The zoom expander 13 is capable of changing the beam diameter of pulsed laser light emitted from the laser light source 2. A controller 46 (to be described later) is capable of adjusting a numerical aperture NA of pulsed laser light emitted from an objective lens 35 (to be described later) to the patient's eye E by changing the beam diameter of the pulsed laser light via the driving of the zoom expander 13. When the beam diameter is increased, the numerical aperture NA is increased, and when the beam diameter is decreased, the numerical aperture NA is decreased.

The high-speed Z scanner 15 (an expander in the embodiment) is provided between the laser light source 2 and the X-Y scanner 25 (specifically, in the embodiment, between the zoom expander 13 and the X-Y scanner 25) on the optical path. As an example, the high-speed Z scanner 15 in the embodiment includes an optical element 16 having negative reflective power, and a high-speed Z scanning drive unit 17 moving the optical element 16 along the optical axis. A lens 21 is provided between the optical element 16 and the X-Y scanner 25. Laser light passing through the high-speed Z scanner 15 is guided to the X-Y scanner 25 by the lens 21.

When the optical element 16 is moved along the optical axis, the concentration position of pulsed laser light is moved in the Z-axis direction. Accordingly, the controller 46 controls the driving of the high-speed Z scanning drive unit 17 such that the optical element 16 is moved, thereby enabling the concentration position to be scanned in the Z-axis direction. The high-speed Z scanner 15 in the embodiment is provided on the upstream side of the X-Y scanner 25 on the optical path. In this case, laser light, which is not scanned in the X-axis direction and Y-axis direction, is incident to the optical element 16. Accordingly, it is easy to reduce the size of the optical element 16 compared to a case where the high-speed Z scanner 15 is provided on the downstream side of the X-Y scanner 25 on the optical path. As a result, the high-speed Z scanner 15 in the embodiment is capable of scanning the concentration position in the Z-axis direction at a higher speed than a Z scanner 44 (to be described later).

The X-Y scanner 25 scans pulsed laser light in an X-Y plane intersecting the optical axis. In the embodiment, the X-Y scanner 25 includes an X-axis deflection device 26 and a Y-axis deflection device 27. The X-axis deflection device 26 scans pulsed laser light, which is emitted from the laser light source 2, in the X-axis direction. The Y-axis deflection device 27 scans the pulsed laser light, which is scanned by the X-axis deflection device 26 in the X-axis direction, in the Y-axis direction. In the embodiment, a galvanometer mirror is adopted as each of the X-axis deflection device 26 and the Y-axis deflection device 27. However, another type of device (scanners such as a polygon mirror and an acousto-optical modulator (AOM)) may be adopted as at least one of the X-axis deflection device 26 and the Y-axis deflection device 27. At least one of the X-axis deflection device 26 and the Y-axis deflection device 27 may include multiple scanners. As an example, in the laser eye surgery apparatus 1 of the embodiment, the X-axis deflection device 26 is formed of two galvanometer mirrors, and thus, a principal ray of pulsed laser light is incident to a predetermined location on the Y-axis deflection device 27 regardless of the amount of scanning in the X-axis direction. As a result, the predetermined location on the Y-axis deflection device 27 becomes a pivot point through which the principal ray of all pulsed laser light rays, which are scanned by the X-Y scanner 25, passes.

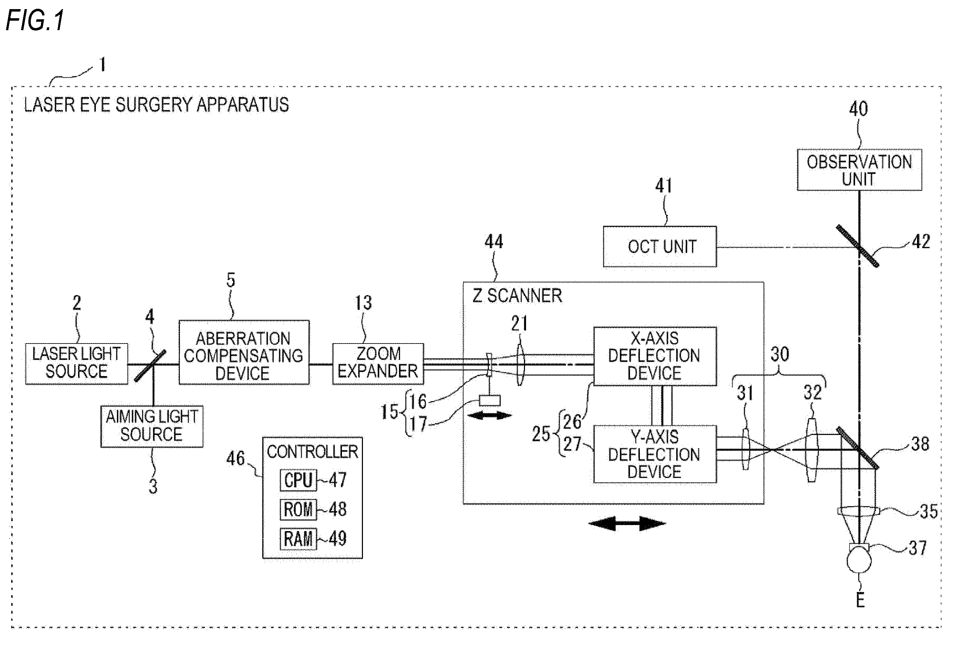

A relay unit 30 is provided between the X-Y scanner 25 and the objective lens 35. The relay unit 30 in the embodiment is a Kepler type relay optical system. Also in a case where a Kepler type relay optical system is formed of three or more optical members, the function of the Kepler type relay optical system can be realized by two lenses. Accordingly, the relay unit 30 is illustrated by two lenses in Fig. 1. When another type of optical element is adopted, the number of optical elements may be different from the number of optical elements which is illustrated. The pivot point in the X-Y scanner 25 (in the embodiment, the predetermined location on a scanning surface of the Y-axis deflection device 27) is conjugated with the objective focal point of the objective lens 35 via an upstream relay optical element 31 and a downstream relay optical element 32 of the relay unit 30. A positional relationship between the upstream relay optical element 31 and the X-Y scanner 25 is held in such a way that the objective focal point of the upstream relay optical element 31 coincides with the pivot point in the X-Y scanner 25. Accordingly, the telecentric performance of pulsed laser light emitted from the upstream relay optical element 31 is maintained.

The objective lens 35 is disposed on the downstream side of the downstream relay optical element 32 of the relay unit 30 on the optical path. Pulsed laser light passing through the objective lens 35 is concentrated in the tissue of the patient's eye E via an eyeball interface 37.

The eyeball interface 37 is connected to and fixed to the patient's eye E. It is possible to adopt various configurations (a liquid immersion interface, a contact lens, and the like) as the configuration of the eyeball interface 37. In a case where a liquid immersion interface is used as the eyeball interface 37, the eyeball interface 37 may include a suction ring and a cap. Negative pressure is applied to the suction ring by a suction pump or the like. As a result, the anterior chamber of the patient's eye E is suctioned by and fixed to the suction ring. The cap covers the circumference of the anterior chamber. During surgery, the cap is filled with liquid (a physiological saline solution, a viscoelastic material having a predetermined index of refraction, or the like) which transmits pulsed laser light. In the embodiment, a material, which transmits measurement light from an OCT unit 41 (to be described later), is used as the material of the eyeball interface 37.

A dichroic mirror 38 is provided between the objective lens 35 and the downstream relay optical element 32 on the optical path. The dichroic mirror 38 in the embodiment reflects a majority of pulsed laser light from the laser light source 2, and a majority of aiming light from the aiming light source 3, and transmits a majority of light from an observation unit 40 (to be described later) and the OCT unit 41. As a result, the optical axes of multiple light rays become coaxial with each other.

The observation unit 40 acquires a front image of the patient's eye E, and a front image of the eyeball interface 37 connected to the patient's eye E. The observation unit 40 in the embodiment is capable of capturing an image of the patient's eye E irradiated with visible light or infrared light, and displaying the image on a monitor (not illustrated). An operator or the like can observe the patient's eye E from the front by watching the monitor.

The OCT unit 41 acquires a tomographic image of the tissue of the patient's eye E, a tomographic image of the eyeball interface 37, and a tomographic image of the liquid with which the cap is filled. As an example, the OCT unit 41 in the embodiment includes a light source; a light splitter; a reference optical system; a scanner; and a detector. The light source emits light required to acquire a tomographic image. The light splitter splits the light emitted from the light source into reference light and measurement light. The reference light is incident to the reference optical system, and the measurement light is incident to the scanner. The reference optical system is configured to change the difference in an optical path length between the measurement light and the reference light. The scanner scans the measurement light on the tissue two-dimensionally. The detector detects a state of coherence between measurement light reflected by the tissue and the reference light passing through the reference optical system. The laser eye surgery apparatus 1 acquires information regarding the depth of the tissue by scanning the measurement light, and detecting a state of coherence between the reflected measurement light and the reference light. The laser eye surgery apparatus 1 acquires a tomographic image of an imaging target (the tissue of the patient's eye E or the like) based on the acquired information regarding the depth.

It is possible to use various configurations as the configuration of the OCT unit 41. Any one of an SS-OCT, an SD-OCT, a TD-OCT, and the like may be adopted as the OCT unit 41. The laser eye surgery apparatus 1 may capture a tomographic image using technology other than optical coherence technology.

In the embodiment, the controller (to be described later) 46 is capable of acquiring the state of the eyeball interface 37 connected to the patient's eye E, based on the tomographic image acquired by the OCT unit 41. As an example, in the embodiment, any one of the following factors is acquired as the state of the eyeball interface 37: the eccentricity, the tilting, and the position of the eyeball interface 37 with respect to the patient's eye E; the distance between the eyeball interface 37 and the objective lens 35; the amount (thickness) of liquid with which the cap is filled; and manufacturing errors of the eyeball interface 37.

The controller 46 in the embodiment is capable of acquiring the shape of the patient's eye E, based on the tomographic image acquired by the OCT unit 41. As an example, in the embodiment, the shape of the cornea (corneal curvature or the like) of the patient's eye E is acquired from a tomographic image.

The laser eye surgery apparatus 1 may acquire the state of the eyeball interface 37 and the shape of the patient's eye E using a different method. For example, the laser eye surgery apparatus 1 may acquire the state of the eyeball interface 37 and the shape of the patient's eye E using at least one of a Scheimpflug image, an SLO image, an image that is captured by the observation unit 40, and the like.

The laser eye surgery apparatus 1 in the embodiment associates the concentration target position of pulsed laser light with a tomographic image of the patient's eye E. As a result, the laser eye surgery apparatus 1 is capable of controlling a pulsed laser light irradiation and scanning operation, and a changing aberration compensation operation of the aberration compensating device 5 using the tomographic image.

A dichroic mirror 42 sets the optical axis of the observation unit 40 to become coaxial with the optical axis of the OCT unit 41. Light passing through the dichroic mirror 42 is set to become coaxial with pulsed laser light, which is emitted from the laser light source 2, by the dichroic mirror 38.

The Z scanner 44 scans the concentration position (spot) of pulsed laser light in the Z-axis direction. As an example, the Z scanner 44 in the embodiment changes the optical path length between the upstream relay optical element 31 and the objective lens 35 by moving an optical unit, which includes the X-Y scanner 25 and the upstream relay optical element 31, along the optical axis. As a result, the spot is scanned in the Z-axis direction. The configuration of the Z scanner 44 can be appropriately changed. For example, the laser eye surgery apparatus 1 may scan the spot in the Z-axis direction by moving at least one of the optical elements (for example, the upstream relay optical element 31, the downstream relay optical element 32, and the objective lens 35), which are positioned on the downstream side of the X-Y scanner 25, in an optical axis direction. The spot can be scanned in the Z-axis direction using only the high-speed Z scanner 15.

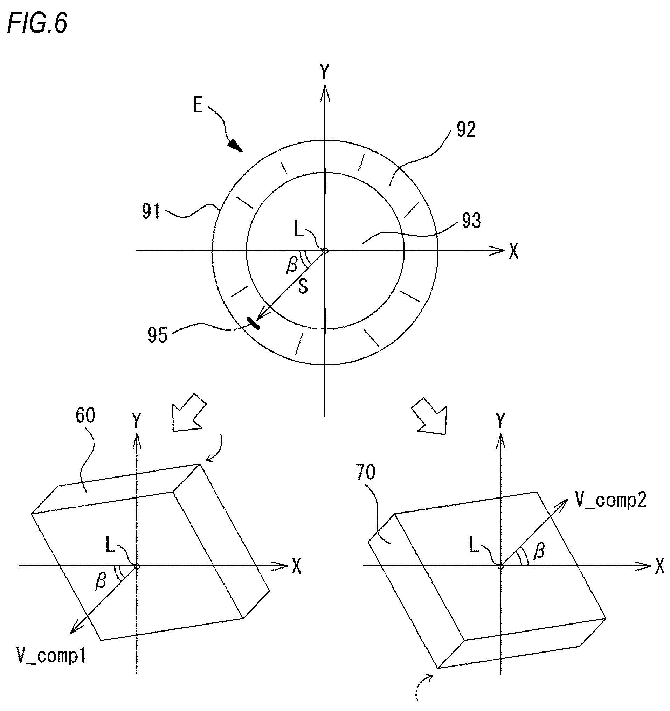

The controller 46 includes a CPU 47; a ROM 48; a RAM 49; a non-volatile memory (not illustrated); and the like. The CPU 47 is in charge of various types of control (the control of the laser light source 2, the control of the operation of the aberration compensating device 5, the control of the operation of the scanners 15, 25, and 44, and the like) of the laser eye surgery apparatus 1. The ROM 48 stores various programs (a compensation processing program required to execute a changing aberration compensation process illustrated in Fig. 6, and the like) required to control the operation of the laser eye surgery apparatus 1, initial values, and the like. The RAM 49 temporarily stores various items of information. The non-volatile memory is a non-transitory storage medium capable of holding stored contents even if the supply of electrical power is shut off. The non-volatile memory may store the compensation processing program and the like.

<Aberration compensating device>

The aberration compensating device 5 will be described with reference to Figs. 2 and 3. As illustrated in Fig. 2, the aberration compensating device 5 in the embodiment includes a lens 51, a lens 52, a first plane-parallel plate 60, a second plane-parallel plate 70, a lens 54, and a lens 55 which are disposed in sequence from the upstream side of the optical axis.

The lenses 51 and 52 converge or diverge pulsed laser light emitted from the laser light source 2 (refer to Fig. 1). As an example, in the embodiment, the lens 51 is a meniscus lens, and diverges and sends pulsed laser light to the lens 52. The lens 52 is a double convex lens, converges the pulsed laser light diverged by the lens 51, and sends the converged pulsed laser light to the plane-parallel plates 60 and 70. The pulsed laser light converged by the lens 51 is in focus between the first plane-parallel plate 60 and the second plane-parallel plate 70, and then is diverged. The configuration of the optical elements positioned on the upstream side of the plane-parallel plates 60 and 70 can be appropriately changed. For example, the number of optical elements is not limited to two. An optical element for diverging pulsed laser light may be disposed between the laser light source 2 and the plane-parallel plates 60 and 70. A mirror may be used to converge or diverge pulsed laser light.

The lenses 54 and 55 make the optical beams of pulsed laser light (passing through the lenses 51 and 52 and the plane-parallel plates 60 and 70) run parallel to each other, and send the pulsed laser light to the downstream optical element. As an example, in the embodiment, the lens 54 is a double convex lens. The pulsed laser light passing through the plane-parallel plates 60 and 70 is converged and sent to the lens 54. The lens 55 is a meniscus lens, and makes the optical beams of pulsed laser light (passing through the lens 54) run parallel to each other. Naturally, the configuration of the lenses 54 and 55 can be changed, similar to the lenses 51 and 52.

The first plane-parallel plate 60 and the second plane-parallel plate 70 are provided in a portion of the optical path of pulsed laser light where the pulsed laser light is converged or diverged. The first plane-parallel plate 60 includes a light incident plane 61 to which pulsed laser light is incident, and a light-emitting plane 62 to which the pulsed laser light is incident from the light incident plane 62, and through which the pulsed laser light is emitted. In the embodiment, the light incident plane 61 is parallel to the light-emitting plane 62. All pulsed laser light rays are not reflected by the inside of the first plane-parallel plate 60, are incident to the light incident plane 61, and are emitted from the light emitting plane. Similar to the first plane-parallel plate 60, the second plane-parallel plate 70 also includes a light incident plane 71 and a light-emitting plane 72 which are parallel to each other.

Various materials ($SiO_2$, $CaF_2$, $BK_7$, and the like) can be adopted as the material of the plane-parallel plates 60 and 70. It is also possible to use a plane-parallel plate including a reflective surface (mirror) by which all pulsed laser rays are reflected. In this case, a light incident plane and a light-emitting plane are the same plane. A wavelength plate may be used as a plane-parallel plate.

It is possible to generate an aberration (to change an aberration) by changing the angle of at least one of the plane-parallel plates provided on the optical path. For example, the thickness and the index of refraction of one plane-parallel plate are respectively assumed to be T and R, and an angle between a normal line perpendicular to the light incident plane 61 and an optical axis L is assumed to be K. In this case, the generated amount of astigmatism A is represented by Expression (1).

$$A = T \cdot K^2 (R^2 - 1)/R^3 \ldots (1)$$

In a case where the numerical aperture is assumed to be sin θ, the generated amount of comma aberration C is represented by Expression (2).

$$C = T \cdot K \cdot \theta (R^2 - 1)/(2R^3) \ldots (2)$$

Accordingly, it is possible to generate an astigmatism and a comma aberration by changing the angle of at least one of the plane-parallel plates. The plane-parallel plates generate an aberration (that is, compensate for an aberration) such that an undesirable aberration caused by the other optical elements (the scanners 25 and 44, the objective lens 35, and the like) is cancelled out and reduced, thereby suppressing an impact of the aberration.

The aberration compensating device 5 in the embodiment includes two plane-parallel plates 60 and 70. In this case, it is possible to compensate for an astigmatism without changing a comma aberration by appropriately tilting the first plane-parallel plate 60 and the second plane-parallel plate 70 with respect to the optical axis L. In addition, it is possible to compensate for an aberration without causing the occurrence of eccentricity (off-axis) of pulsed laser light by appropriately tilting the first plane-parallel plate 60 and the second plane-parallel plate 70 with respect to the optical axis L.

The eccentricity (off-axis) implies that the optical path of pulsed laser light after passing through the two plane-parallel plates 60 and 70 is offset from an optical path when the angle formed between the two plane-parallel plates 60 and 70 (that is, the angle formed between the light incident planes 61 and 71 and the light-emitting planes 62 and 72) is set to an initial angle (to be described later).

The initial angle is the angle of each of the plane-parallel plates 60 and 70 with respect to the optical axis L in a state where an aberration of a compensation target is not yet compensated for. For example, in a case where an aberration of a compensation target occurs due to manufacturing errors, the initial angle is an angle at which the respective light incident planes 61 and 71 and the respective light-emitting planes 62 and 72 of the plane-parallel plate 60 and 70 are perpendicular to the optical axis L of pulsed laser light. In a case where an aberration (for example, an aberration changed by the scanning of pulsed laser light) occurring due to other factors is dynamically compensated for in a state where the aberration occurring due to the manufacturing errors is already compensated for, the initial angle is an angle which is set to compensate for only the aberration occurring due to the manufacturing errors.

Particularly, in the embodiment, the two plane-parallel plates 60 and 70 have the same index of refraction and the same thickness. In this case, as illustrated in Fig. 2, when the two plane-parallel plates 60 and 70 are tilted at the same amount of tilt (an angle α in Fig. 2) in opposite directions, an astigmatism is compensated for without the occurrence of eccentricity. Accordingly, the angle of the two plane-parallel plates 60 and 70 can be more simply set.

As illustrated in Fig. 3, the laser eye surgery apparatus 1 in the embodiment includes a holding mechanism 80 holding the first plane-parallel plate 60. Since a holding mechanism holding the second plane-parallel plate 70 may adopt the same configuration as that of the holding mechanism 80 illustrated in Fig. 3, a description of the holding mechanism will be omitted. The holding mechanism 80 holds the first plane-parallel plate 60 in such a way that the angle of the light incident plane 61 and the light-emitting plane 62 of the first plane-parallel plate 60 can be changed with respect to the optical axis L of pulsed laser light. The term "the change of an angle" includes both of a change in the "amount of tilt" of the first plane-parallel plate 60 with respect to the optical axis L and a change in the "direction" of a normal line of the first plane-parallel plate 60 with respect to the optical axis L.

As an example, the holding mechanism 80 in the embodiment includes a first movable holding portion 81; a second movable holding portion 85; and a fixation holding portion 89. The first movable holding portion 81 includes a connecting piece 82 and a drive unit 83. The connecting piece 82 is connected to a portion (a left upper portion in the example illustrated in Fig. 3) of the first plane-parallel plate 60. The drive unit 83 moves the connecting piece 82 in the Z-axis direction. Various actuators such as a motor and a solenoid can be adopted as the drive unit 83. The second movable holding portion 85 includes a connecting piece 86 and a drive unit 87. The connecting piece 86 is connected to a portion (a right lower portion in the example illustrated in Fig. 3) of the first plane-parallel plate 60, which is different from the portion to which the connecting piece 82 is connected. The drive unit 87 moves the connecting piece 86 in the Z-axis direction. The fixation holding portion 89 fixes a portion (a right upper portion in the example illustrated in Fig. 3) of the first plane-parallel plate 60, which is different from the portions to which the connecting pieces 82 and 86 are respectively connected. Specifically, the fixation holding portion 89 fixes the position of a portion of the first plane-parallel plate 60 in such a way that the angle of the first plane-parallel plate 60 can be changed.

The controller 46 is capable of freely changing the angle of the first plane-parallel plate 60 by controlling the driving of the drive units 83 and 87. In the example illustrated in Fig. 3, in a case where the angle of the first plane-parallel plate 60 (specifically, the light incident plane 61 and the light-emitting plane 62 of the first plane-parallel plate 60) is set to the initial angle, the first plane-parallel plate 60 is perpendicular to the optical axis L. In this case, a normal direction V_int of the light incident plane 61 of the first plane-parallel plate 60 coincides with the direction of the optical axis L. When the drive unit 83 moves the connecting piece 82 in a positive Z-axis direction, and the drive unit 87 moves the connecting piece 86 in a negative Z-axis direction from this state, a normal direction V_comp (that is, the direction of a normal line extending to the upstream side of the optical path from the light incident plane 61) of the light incident plane 61 is tilted to a left upper side in Fig. 3 (a state illustrated by the dotted line in Fig. 3). For example, when only the connecting piece 86 is moved in the positive Z-axis direction, the normal direction of the light incident plane 61 is tilted to the lower side in Fig. 3.

The position of disposition of the aberration compensating device 5 will be described. As illustrated in Fig. 1, in the embodiment, the aberration compensating device 5 including the plane-parallel plates 60 and 70 is disposed on the upstream side (that is, close to the laser light source 2) of the objective lens 35 on the optical path of pulsed laser light from the laser light source 2 to the patient's eye E. In this case, the distance between the objective lens 35 and the patient's eye E is easily reduced. Accordingly, the laser eye surgery apparatus 1 is capable of irradiating the patient's eye E with laser light at the high numerical aperture NA without using the excessively large objective lens 35. In a case where the aberration compensating device 5 is disposed on the upstream side of the objective lens 35 on the optical path, the size of the aberration compensating device 5 can be easily reduced compared to a case where the aberration compensating device 5 is disposed on the downstream side of the objective lens 35.

<Typical Aberration Compensation Method>

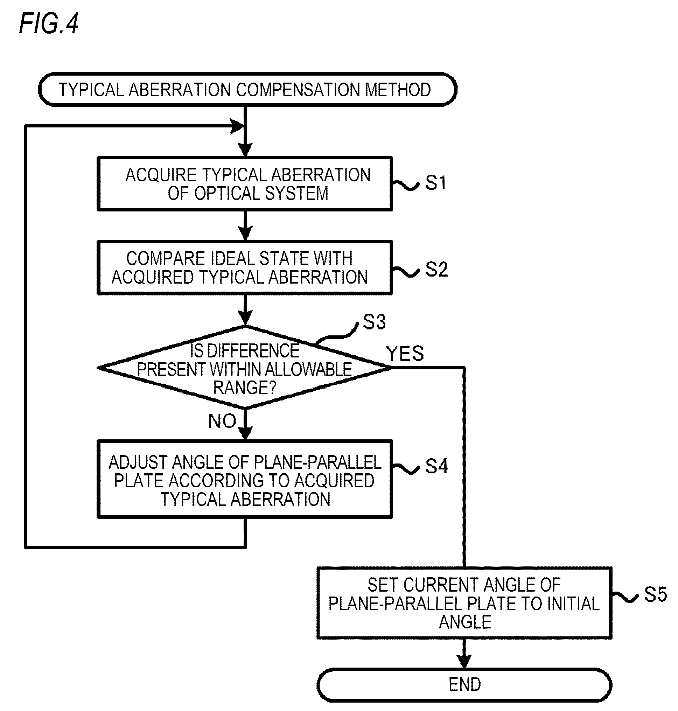

A typical aberration compensation method in the embodiment will be described with reference to Fig. 4. A typical aberration refers to an undesirable aberration which may typically occur due to manufacturing errors, usage degradation, or the like regardless of a change in the spot (concentration position) caused by the driving of the scanners 25 and 44. The typical aberration compensation method illustrated in Fig. 4 can be executed when the operations of the laser eye surgery apparatus 1 during manufacturing are checked, or maintenance is performed outside of the factory by a manufacturer's employee or the like. In a case exemplarily described in the embodiment, a worker manually adjusts the respective angles of the plane-parallel plates 60 and 70. However, the controller 46 controls the drive units 83 and 87 such that the typical aberration compensation method illustrated in Fig. 4 can be executed.

First, a worker acquires a typical aberration of an optical system which occurs due to manufacturing errors or usage degradation (S1). The worker may acquire an aberration, which typically occurs in the optical system, by using an apparatus (a wavefront sensor, a beam profiler, or the like) capable of observing and measuring an aberration. Subsequently, the worker compares an ideal state, in which a typical aberration does not occur, with the typical aberration acquired in step S1 (S2). The worker determines whether or not the difference therebetween acquired by the comparison in step S2 is present within an allowable range (S3). The allowable range may be determined in advance, or may be determined by the worker.

When the difference is not present within the allowable range (NO in step S3), the worker adjusts the respective angles of the plane-parallel plates 60 and 70 according to the typical aberration acquired in step S 1 such that the typical aberration is reduced (S4). Steps S1 to S4 are repeated until the typical aberration becomes within the allowable range. When the typical aberration is present within the allowable range (YES in step S3), the worker sets the current angle of each of the plane-parallel plates 60 and 70 to an initial angle at which the typical aberration is compensated for (S5), and ends the operation. In step S5, the non-volatile memory or the like may store the respective positions of the connecting pieces 82 and 86 in a state where the plane-parallel plates 60 and 70 are set to be at the initial angle. After untightening the fixing of the plane-parallel plates 60 and 70 to the holding mechanism 80, and performing step S4, the worker may set an initial angle by fixing the plane-parallel plates 60 and 70 to the holding mechanism 80 in step S5.

<Changing Aberration Compensation Process>

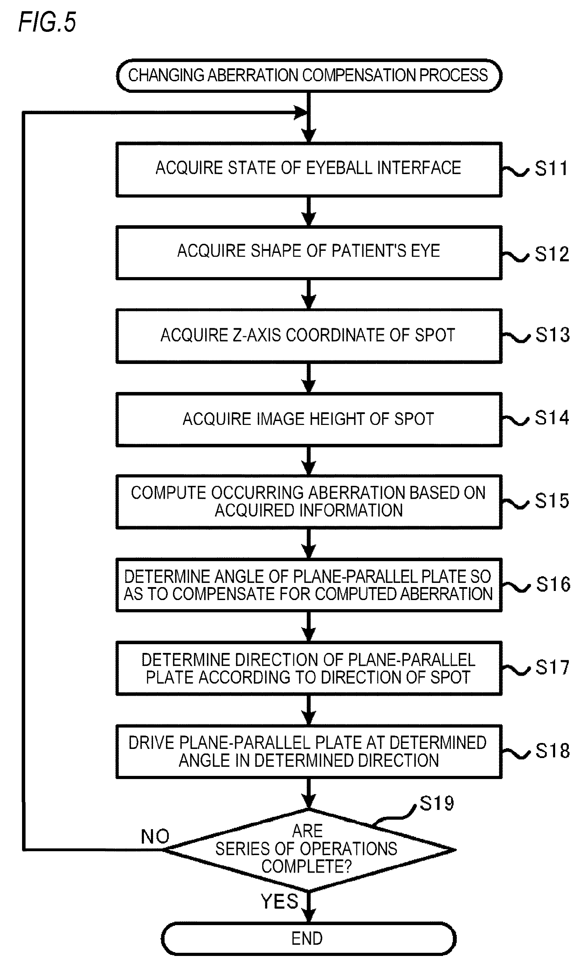

A changing aberration compensation process executed by the laser eye surgery apparatus 1 in the embodiment will be described with reference to Figs. 5 and 6. A changing aberration refers to an aberration which is changed according to an operation condition. In the embodiment, a changing aberration may occur due to the state of the eyeball interface 37 connected to the patient's eye E, the position of a spot scanned by the scanners 25 and 44, the direction of a spot with respect to the optical axis L, the position of the patient's eye E, and the shape of the patient's eye E. Accordingly, the laser eye surgery apparatus 1 in the embodiment executes the changing aberration compensation process illustrated in Fig. 5 so as to compensate for this changing aberration. Naturally, the laser eye surgery apparatus 1 is capable of compensating for only a portion of multiple changing aberrations via the changing aberration compensation process.

In a case where an instruction is input to indicate a start of a series of operations using pulsed laser light, the changing aberration compensation process illustrated in Fig. 5 is executed by the CPU (processor) 47 of the controller 46. Particularly, in the embodiment, the changing aberration compensation process is executed during an operation in which a corneal port in the patient's eye E is formed. The corneal port is an incision having a circular shape in a front view which is formed in the vicinity of an outer circumferential end portion of the cornea, and through which an operation instrument is inserted into the patient's eye E. An aberration is likely to occur due to an impact of a corneal curvature in a laser operation in which a corneal port is formed. Since an image height, which is a distance between the corneal port and the optical axis L, is large, an aberration is likely to be increased. In a case where the liquid immersion eyeball interface 37 is used where the corneal is not applanated by a contact lens, an aberration may occur due to a variation in the corneal curvatures of the patients' eyes E, a variation in the distance between the apparatus and the cornea, and the like. The laser eye surgery apparatus 1 in the embodiment is capable of suppressing an impact of an aberration, and appropriately forming a corneal port by executing the changing aberration compensation process during the formation of the cornea port.

A time the changing aberration compensation process is executed is not limited to when the corneal port is formed. For example, the laser eye surgery apparatus 1 may execute the changing aberration compensation process during execution of a limbal relaxing incision (LRI). An aberration may also occur during the execution of the LRI due to the same reasons as during the formation of a corneal port. The changing aberration compensation process can also be executed during incision of an anterior lens capsule.

First, the controller 46 acquires the state of the eyeball interface 37 connected to the patient's eye E (S11). As described above, the controller 46 in the embodiment is capable of acquiring any one of the following factors as the state of the eyeball interface 37, based on the tomographic image captured by the OCT unit 41 (refer to Fig. 1): the eccentricity, the tilting, the position, and the distance of the eyeball interface 37; the amount of liquid; and manufacturing errors.

The controller 46 acquires the shape of the patient's eye E (S12). As described above, the controller 46 in the embodiment acquires the shape (more specifically, the corneal curvature and the like) of the cornea of the patient's eye E, based on the tomographic image captured by the OCT unit 41.

The controller 46 acquires the Z-axis coordinate of a spot (target position) on which pulsed laser light is concentrated (S13). The controller 46 acquires the image height (distance between the spot and the optical axis L in the X-Y plane) of the spot (S14). In the embodiment, control data used to control the driving of the scanners 25 and 44 is prepared in advance. The operator concentrates pulsed laser light on each spot, and performs an operation by controlling the driving of the scanners 25 and 44 according to the control data via the controller 46. The controller 46 is capable of acquiring the Z-axis coordinate and the image height of the spot with reference to the control data and the like.

The controller 46 computes an aberration, which occurs when pulsed laser light is concentrated on the target position, based on items of information acquired in steps S11 to S14 (S15). An aberration can be computed in various methods. For example, a table or a computational expression, in which each parameter acquired in step S11 to S 14 is associated with the amount of aberration occurring in the optical system, may be prepared in advance, based on experiment results.

In order to compensate for the aberration computed in step S15, the controller 46 determines the amount of tilt angle of each of the plane-parallel plates 60 and 70 with respect to the optical axis L, based on the computed amount of aberration (S16). The amount of tilt can be determined in various methods. For example, a table or a computational expression, in which the computed amount of aberration is associated with the amount of tilt angle of each of the plane-parallel plates 60 and 70 with respect to the optical axis L, may be prepared in advance, based on experiment results. It is also possible to directly determine the respective angles of the plane-parallel plates 60 and 70 from the parameters acquired in steps S11 to S 14 without computing an aberration occurring in the optical system.

The controller 46 determines the direction (that is, a direction in which the normal direction of each of the plane-parallel plates 60 and 70 extends with respect to the optical axis L in the X-Y plane) of each of the plane-parallel plates 60 and 70, according to the direction in which the spot is located with respect to the optical axis L in the X-Y plane (S17).

In a case where an astigmatism is compensated for without changing a comma aberration, a relationship between the direction of a spot with respect to the optical axis L and the direction of each of the plane-parallel plates 60 and 70 will be described with reference to Fig. 6. Fig. 6 shows views when the position of a corneal port 95 formed in the patient's eye E is seen from the direction of the optical axis L (that is, the front of the patient's eye E). The optical axis L coincides with a Z axis. When the patient's eye E is seen from the front, an iris 92 and a pupil 93 inside of an outer circumference 91 of the cornea are observed. As described above, the corneal port 95 is formed in the vicinity of the outer circumferential end portion of the cornea. In the example illustrated in Fig. 6, the corneal port 95 is positioned on a left lower side of the optical axis L in a front view. Specifically, the amount of tilt angle formed between an X axis and a direction S of the corneal port 95 in the X-Y plane with respect to the optical axis L is β degrees.

A left lower view in Fig. 6 illustrates the first plane-parallel plate 60 when seen from the direction of the optical axis L, which is tilted during the formation of the corneal port 95. In the embodiment, the amount of tilt angle formed between the X axis and a normal direction V_comp1 (the direction of the normal line extending to the upstream side) of the light incident plane 61 of the first plane-parallel plate 60 when seen from the X-Y plane is β degrees. The amount of tilt angle is the same as the amount of the tilt angle formed between the X axis and the direction S of the corneal port 95 with respect to the optical axis L. When the first plane-parallel plate 60 is seen from the X-Y plane, the normal direction V_comp1 is the same as the direction S of the corneal port 95.

A right lower view in Fig. 6 illustrates the second plane-parallel plate 70 when seen from the direction of the optical axis L, which is tilted during the formation of the corneal port 95. In the embodiment, the amount of tilt angle formed between the X axis and a normal direction V_comp2 of the light incident plane 71 of the second plane-parallel plate 70 when seen from the X-Y plane is β degrees. When the second plane-parallel plate 70 is seen from the X-Y plane, the normal direction V_comp2 is opposite to the direction S of the corneal port 95.

As illustrated in Fig. 2, in a case where an astigmatism is compensated for without changing a comma aberration, the controller 46 tilts the two plane-parallel plates 60 and 70 at the same amount of tilt angle (α degrees in the example illustrated in Fig. 2) with respect to the initial angle (in the embodiment, an angle perpendicular to the optical axis L).

That is, in the embodiment, in a case where an astigmatism is compensated for without changing a comma aberration by tilting the two plane-parallel plates 60 and 70 from the initial angle, the controller 46 tilts the two plane-parallel plates 60 and 70 at the same amount of tilt angle in opposite directions. In this case, pulsed laser light is offset from the optical path. Accordingly, the controller 46 is capable of controlling irradiation of pulsed laser light without taking eccentricity into consideration.

A description will be given referring to Fig. 5. When determining the amount of the tilt angle and the direction of each of the plane-parallel plates 60 and 70 (S16 and S17), the controller 46 drives the plane-parallel plates 60 and 70 by the determined amount of the tilt angle in the determined direction (S18). Steps S11 to S18 are repeated until the series of operations are complete (NO in step S19). When the series of operations are complete (YES in step S19), the changing aberration compensation process ends.

As described above, the laser eye surgery apparatus 1 in the embodiment includes the plane-parallel plates 60 and 70 disposed on the optical path of pulsed laser light from the laser light source 2 to the patient's eye E. The plane-parallel plate 60 is a member in which the light incident plane 61 to which pulsed laser light is incident, and the light-emitting plane 62 emitting the pulsed laser light incident from the light incident plane 61 are parallel to each other (or are the same plane). The plane-parallel plate 70 is a member in which the light incident plane 71 to which pulsed laser light is incident, and the light-emitting plane 72 emitting the pulsed laser light incident from the light incident plane 71 are parallel to each other (or are the same plane). When the plane-parallel plates 60 and 70 are provided in a region in which pulsed laser light is converged or diverged, an aberration may occur according to the angle between the optical axis of the pulsed laser light and each of the plane-parallel plates 60 and 70. Accordingly, the laser eye surgery apparatus 1 in the embodiment is capable of appropriately suppressing an impact of an aberration by generating an aberration (that is, compensating for an undesirable aberration) via the plane-parallel plates 60 and 70 so as to cancel out and reduce the undesirable aberration.

It is also considered that another method (for example, a method in which a lens provided on an optical path is moved in an optical axis direction) is used to compensate for an aberration. However, when the lens is moved in the optical axis direction, not only an aberration may be changed but also the concentration position of pulsed laser light may be changed in the Z-axis direction. In contrast, while suppressing a change in the concentration position in the Z-axis direction, the laser eye surgery apparatus 1 in the embodiment is capable of suppressing an impact of an aberration by using the plane-parallel plates 60 and 70. In some cases, it is also possible to compensate for only at least one of an astigmatism and a comma aberration among various aberrations by using the plane-parallel plates 60 and 70.

The laser eye surgery apparatus 1 in the embodiment includes the holding mechanism 80 holding the plane-parallel plates 60 and 70. The angle (that is, the direction, and the amount of tilt angle) of each of the plane-parallel plates 60 and 70, which are held by the holding mechanism 80, with respect to the optical axis of pulsed laser light can be changed. Accordingly, in the embodiment, an impact of an aberration is more appropriately suppressed by adjusting the respective angles of the plane-parallel plates 60 and 70 according to an occurring aberration.

The laser eye surgery apparatus 1 in the embodiment includes the drive units 83 and 87 driving the holding mechanism 80 of each of the plane-parallel plates 60 and 70, and the controller 46 controlling the drive units 83 and 87. Accordingly, the laser eye surgery apparatus 1 is capable of easily changing the respective angles of the plane-parallel plates 60 and 70 according to whether or not compensation of an aberration is required. In a case where an aberration is changed during an operation, the controller 46 can appropriately change the respective angles of the plane-parallel plates 60 and 70 according to the changing aberration during the operation.

An aberration is changed according to the position (that is, at least one of an image height and the depth of a spot) of a spot, or the shape of the patient's eye E. The laser eye surgery apparatus 1 in the embodiment adjusts the amount of tilt angle of each of the plane-parallel plates 60 and 70 with respect to the optical axis L, according to at least one of the position of the spot and the shape of the patient's eye E. Accordingly, the laser eye surgery apparatus 1 is capable of appropriately adjusting the respective angles of the plane-parallel plates 60 and 70 according to the occurring aberration.

When the direction of the normal line (extending in the X-Y plane) of each of the plane-parallel plates 60 and 70 is changed, the mode of an aberration is also changed. The laser eye surgery apparatus 1 in the embodiment changes the direction of a normal line (extending in the X-Y plane) of each of the plane-parallel plates 60 and 70 according to the direction of a spot in the X-Y plane with respect to the optical axis L. Accordingly, the laser eye surgery apparatus 1 is capable of more appropriately compensating for an aberration by generating an aberration according to the position of the spot in the X-Y plane via the plane-parallel plates 60 and 70.

The laser eye surgery apparatus 1 in the embodiment drives the plane-parallel plates 60 and 70 according to the state of the eyeball interface 37 connected to the patient's eye E. Accordingly, the laser eye surgery apparatus 1 is capable of more appropriately suppressing an impact of an aberration even in a case where an aberration is changed by the eyeball interface 37.

The laser eye surgery apparatus 1 in the embodiment includes the two plane-parallel plates 60 and 70. For example, while a comma aberration is not changed, an astigmatism is appropriately compensated for by tilting the first plane-parallel plate 60 and the second plane-parallel plate 70 at the same amount of tilt angle from the initial angle in the opposite direction. In this case, even if the angle of each of the two plane-parallel plates 60 and 70 is changed, pulsed laser light passing through the plane-parallel plates 60 and 70 passes through the same optical path as when the angle of each of the two plane-parallel plates 60 and 70 is not changed and is set to the initial angle (that is, the pulsed laser light is offset from the optical path). The laser eye surgery apparatus 1 is also capable of compensating for both of an astigmatism and a comma aberration by changing the respective angles of the two plane-parallel plates 60 and 70 from the initial angle to different angles (for example, by tilting only one plane-parallel plate). The laser eye surgery apparatus 1 is also capable of compensating for a diagonal astigmatism by tilting the two plane-parallel plates 60 and 70 in directions which are not opposite to each other.

The plane-parallel plates 60 and 70 in the embodiment are disposed closer to the laser light source 2 than (disposed on the upstream side of) the objective lens 35 on the optical path of pulsed laser light. In this case, the distance between the objective lens 35 and the patient's eye E is easily reduced. Accordingly, an operator can perform a laser operation using the high numerical aperture NA via the laser eye surgery apparatus 1 in the embodiment even if the excessively large objective lens 35 is not used. In this case, the size of each of the plane-parallel plates 60 and 70 can also be easily reduced.

The contents disclosed in the embodiment are simply an example. Accordingly, the contents disclosed in the embodiment can also be changed. For example, in the embodiment, the two plane-parallel plates 60 and 70 are used. However, the number of plane-parallel plates used can also be changed. For example, the number of plane-parallel plates may be one insofar as the one plane-parallel plate is effective in changing an astigmatism and a comma aberration. The laser eye surgery apparatus 1 may include three or more plane-parallel plates. In this case, it is possible to freely compensate for each of an astigmatism and a comma aberration. For example, it is also possible to compensate for an astigmatism without changing a comma aberration by tilting two of the three or more plane-parallel plates at the same amount of a tilt angle in opposite directions, and setting the respective angles of the other plane-parallel plates to be perpendicular to the optical axis L. It is also possible to compensate for only a comma aberration by appropriately tilting one of three plane-parallel plates, and tilting the other two plane-parallel plates in opposite directions. The laser eye surgery apparatus 1 may include four or more plane-parallel plates. In this case, regardless of the position of a spot in the X-Y plane, an aberration is compensated for by a simple configuration in which the rotational axes of two plane-parallel plates are disposed in the X-axis direction, and the rotational axes of the other two plane-parallel plates are placed in a Y-axis direction.

The configuration for changing the angle of the plane-parallel plate can also be appropriately changed. For example, the amount of the tilt angle and the direction of a plane-parallel plate are freely changed by using a configuration in which the plane-parallel plate is rotated around the optical axis L while being rotated around one axis perpendicular to the optical axis L. The laser eye surgery apparatus 1 may rotate a plane-parallel plate in multiple directions around each of multiple axes (the center of rotation) perpendicular to the optical axis L.

In the embodiment, the angle of each of the plane-parallel plates 60 and 70 is changed by controlling the drive units 83 and 87 via the controller 46. However, the drive unit changing the angle of the plane-parallel plate may not be provided, and a worker or the like may manually change the angle of the plane-parallel plate. For example, in a case where an appropriate change in the angle of the plane-parallel plate is not required during an operation, the configuration of the apparatus is simplified by adopting a configuration in which the angle of plane-parallel plate is manually changed.

The laser eye surgery apparatus 1 in the embodiment adjusts the respective angles of the plane-parallel plates 60 and 70 according to the position of a spot, the shape of the patient's eye E, and the state of the eyeball interface 37. However, the method of adjusting the respective angles of plane-parallel plates 60 and 70 can also be changed. For example, the laser eye surgery apparatus 1 may adjust the respective angles of the plane-parallel plates 60 and 70 based on only a portion of the position of a spot, the shape of the patient's eye E, and the state of the eyeball interface 37. In a case where the state of the eyeball interface 37 is taken into consideration, the laser eye surgery apparatus 1 may adjust the respective angles of the plane-parallel plates 60 and 70 based on only a portion of eccentricity, tilting, the thickness of the liquid, and manufacturing errors, or may adjust the respective angles of the plane-parallel plates 60 and 70 according to the type of the eyeball interface 37.

The laser eye surgery apparatus 1 in the embodiment is capable of linearly adjusting the respective angles of the plane-parallel plates 60 and 70 according to an aberration to be compensated for. However, the laser eye surgery apparatus 1 may adjust the angle of the plane-parallel plate in a stepwise manner. The laser eye surgery apparatus 1 may determine an angle required to compensate for a changing aberration in advance, instead of computing the angle of the plane-parallel plate according to the amount of occurring aberration. That is, the laser eye surgery apparatus 1 may adjust the angle of the plane-parallel plate without feedback information on the amount of aberration. Also, in this case, an impact of the aberration is suppressed.

In the embodiment, the two plane-parallel plates 60 and 70 have the same index of refraction and the same thickness. Accordingly, while the occurrence of eccentricity is suppressed, an astigmatism is compensated for by rotating the two plane-parallel plates 60 and 70 at the same amount of tilt angle from the initial angle in the opposite directions. However, multiple plane-parallel plates are not necessarily required to have the same index of refraction and the same thickness. Pulsed laser light may be offset from the optical path due to a change in the angle of one or multiple plane-parallel plates. Also, in this case, it is possible to appropriately concentrate pulsed laser light to a target position by controlling the driving of the scanners 25 and 44 via the controller 46 while taking the eccentricity into consideration.

**Claims**

1. A laser eye surgery apparatus comprising:

   a laser light source configured to emit pulsed laser light;
   a scanner configured to scan the pulsed laser light emitted from the laser light source;
   a condenser configured to concentrate the pulsed laser light scanned by the scanner in a tissue of a patient's eye; and
   a plane-parallel plate which is provided in a region in which the pulsed laser light is converged or diverged on an optical path of the pulsed laser light from the laser light source to the patient's eye,
   wherein the plane-parallel plate has a light incident plane to which the pulsed laser light is incident, and a light-emitting plane emitting the pulsed laser light incident from the light incident plane, and
   wherein the light incident plane and the light-emitting plane are parallel to each other, or are the same plane.

2. The laser eye surgery apparatus according to claim 1 further comprising a holding mechanism configured to hold the plane-parallel plate to change an angle of the plane-parallel plate with respect to an optical axis of the pulsed laser light.

3. The laser eye surgery apparatus according to claim 2, further comprising:

   a drive unit configured to change the angle of the plane-parallel plate with respect to the optical axis by driving the holding mechanism; and
   a controller configured to control the drive unit to a change in the angle of the plane-parallel plate.

4. The laser eye surgery apparatus according to claim 3, wherein the controller adjusts amount of a tilt angle of the plane-parallel plate with respect to the optical axis according to at least one of a position of a spot on which the pulsed laser light is concentrated, and a shape of the patient's eye.

5. The laser eye surgery apparatus according to claim 3 or 4, wherein the controller changes a direction in which a normal line of the plane-parallel plate extends with respect to an X-Y plane perpendicular to the optical axis, according to a direction in which a spot on which the pulsed laser light is concentrated is located with respect to the optical axis in the X-Y plane.

6. The laser eye surgery apparatus according to any one of claims 3 to 5, wherein the controller controls the drive unit to change the angle of the plane-parallel plate according to a state of an eyeball interface connected to the patient's eye.

7. The laser eye surgery apparatus according to any one of claims 1 to 6, wherein two or more plane-parallel plates are provided in the laser eye surgery apparatus.

8. The laser eye surgery apparatus according to any one of claims 3 to 6, wherein
   two plane-parallel plates are provided in the laser eye surgery apparatus, and
   the controller drive the drive unit to tilt the two plane-parallel plates at same amount of a tilt angle from an initial angle in opposite directions.

9. The laser eye surgery apparatus according to any one of claims 1 to 8, wherein
   the condenser includes an objective lens configured to concentrate the pulsed laser light scanned by the scanner, and

the plane-parallel plate is disposed closer to the laser light source than the objective lens on the optical path of the pulsed laser light from the laser light source to the patient's eye.

10. An aberration compensation method by which an aberration of a laser eye surgery apparatus is compensated, wherein the laser eye surgery apparatus includes: a laser light source configured to emit pulsed laser light; a scanner configured to scan the pulsed laser light emitted from the laser light source; a condenser configured to concentrate the pulsed laser light scanned by the scanner in a tissue of a patient's eye; and a plane-parallel plate which is provided in a region in which the pulsed laser light is converged or diverged on an optical path of the pulsed laser light from the laser light source to the patient's eye, wherein the plane-parallel plate has a light incident plane to which the pulsed laser light is incident, and a light-emitting plane emitting the pulsed laser light incident from the light incident plane, and wherein the light incident plane and the light-emitting plane are parallel to each other, or are the same plane, wherein the aberration compensation method comprises:

acquiring an aberration which occurs in an optical system of the laser eye surgery apparatus due to manufacturing errors or usage degradation; and
adjusting the angle of the plane-parallel plate with respect to an optical axis of the pulsed laser light according to the acquired aberration.

FIG.1

EP 3 061 430 A1

FIG.2

EP 3 061 430 A1

FIG.3

FIG.4

```
┌─────────────────────────────────────────────────┐
│   TYPICAL ABERRATION COMPENSATION METHOD         │
└─────────────────────────────────────────────────┘
                         │
       ┌─────────────────┴───────────┐
       │                             ▼
       │        ┌──────────────────────────────┐
       │        │   ACQUIRE TYPICAL ABERRATION  │ ∿ S1
       │        │      OF OPTICAL SYSTEM         │
       │        └──────────────────────────────┘
       │                    │
       │                    ▼
       │        ┌──────────────────────────────┐
       │        │  COMPARE IDEAL STATE WITH     │ ∿ S2
       │        │  ACQUIRED TYPICAL ABERRATION  │
       │        └──────────────────────────────┘
       │                    │           S3
       │                    ▼        ◢
       │              ╱──────────────╲        YES
       │           ╱   IS DIFFERENCE   ╲────────────┐
       │          ◁ PRESENT WITHIN ALLOWABLE▷       │
       │           ╲     RANGE?        ╱            │
       │              ╲──────────────╱              │
       │                  NO │                      │
       │                     ▼                      │
       │        ┌──────────────────────────────┐    │
       │        │ ADJUST ANGLE OF PLANE-PARALLEL│ ∿ S4 │
       │        │ PLATE ACCORDING TO ACQUIRED   │    │
       │        │    TYPICAL ABERRATION         │    │
       │        └──────────────────────────────┘    │
       │                     │                      │
       └─────────────────────┘                      │
                                                    ▼
                              ┌──────────────────────────────┐
                              │    SET CURRENT ANGLE OF       │ ∿ S5
                              │ PLANE-PARALLEL PLATE TO INITIAL│
                              │          ANGLE                │
                              └──────────────────────────────┘
                                         │
                                         ▼
                              ┌──────────────────────────────┐
                              │            END               │
                              └──────────────────────────────┘
```

*FIG.5*

```
┌──────────────────────────────────────────────────┐
│    CHANGING ABERRATION COMPENSATION PROCESS        │
└──────────────────────────────────────────────────┘
                         │
                         ▼
┌──────────────────────────────────────────────────┐
│        ACQUIRE STATE OF EYEBALL INTERFACE          │  S11
└──────────────────────────────────────────────────┘
                         │
                         ▼
┌──────────────────────────────────────────────────┐
│          ACQUIRE SHAPE OF PATIENT'S EYE            │  S12
└──────────────────────────────────────────────────┘
                         │
                         ▼
┌──────────────────────────────────────────────────┐
│         ACQUIRE Z-AXIS COORDINATE OF SPOT          │  S13
└──────────────────────────────────────────────────┘
                         │
                         ▼
┌──────────────────────────────────────────────────┐
│            ACQUIRE IMAGE HEIGHT OF SPOT            │  S14
└──────────────────────────────────────────────────┘
                         │
                         ▼
┌──────────────────────────────────────────────────┐
│    COMPUTE OCCURRING ABERRATION BASED ON           │  S15
│           ACQUIRED INFORMATION                     │
└──────────────────────────────────────────────────┘
                         │
                         ▼
┌──────────────────────────────────────────────────┐
│  DETERMINE ANGLE OF PLANE-PARALLEL PLATE SO        │  S16
│ AS TO COMPENSATE FOR COMPUTED ABERRATION           │
└──────────────────────────────────────────────────┘
                         │
                         ▼
┌──────────────────────────────────────────────────┐
│   DETERMINE DIRECTION OF PLANE-PARALLEL            │  S17
│  PLATE ACCORDING TO DIRECTION OF SPOT              │
└──────────────────────────────────────────────────┘
                         │
                         ▼
┌──────────────────────────────────────────────────┐
│  DRIVE PLANE-PARALLEL PLATE AT DETERMINED          │  S18
│     ANGLE IN DETERMINED DIRECTION                  │
└──────────────────────────────────────────────────┘
                         │
                         ▼
                    ◇ S19
              ARE
NO      SERIES OF OPERATIONS
           COMPLETE?
                   YES
                    │
                    ▼
┌──────────────────────────────────────────────────┐
│                      END                           │
└──────────────────────────────────────────────────┘
```

FIG.6

**EUROPEAN SEARCH REPORT**

Europäisches
Patentamt
European
Patent Office
Office européen
des brevets

Application Number

EP 16 15 7534

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/053367 A1 (WAVELIGHT GMBH [DE]; GORSCHBOTH CLAUDIA [DE]; VOGLER KLAUS [DE]; DONIT) 18 April 2013 (2013-04-18) | 1 | INV. A61F9/008 |
| Y | * abstract * <br> * page 13, line 1 - page 15, line 6 * <br> * figure 4a * <br> ----- | 2-5,7,9 | |
| Y | US 2009/231692 A1 (YOSHIDA YUKI [JP] ET AL) 17 September 2009 (2009-09-17) | 2-5,7,9 | |
| A | * paragraphs [0034], [0036], [0037], [0076] * <br> * claim 4 * <br> * figure 4 * <br> ----- | 6,8,10 | |
| E | WO 2016/105966 A1 (NOVARTIS AG [CH]; RAKSI FERENC [US]) 30 June 2016 (2016-06-30) <br> * abstract * <br> * paragraphs [0049], [0057], [0065] * <br> * figures 4,6 * <br> ----- | 1,9 | |
| X | US 2007/103642 A1 (BILLE JOSEF [DE]) 10 May 2007 (2007-05-10) <br> * abstract * <br> * paragraphs [0012], [0016], [0021] * <br> * figures 1,5 * <br> ----- | 1,7 | TECHNICAL FIELDS SEARCHED (IPC) <br><br> A61F |

The present search report has been drawn up for all claims

| Place of search | Date of completion of the search | Examiner |
|---|---|---|
| Munich | 13 July 2016 | Jansen, Birte |

## EP 3 061 430 A1

## ANNEX TO THE EUROPEAN SEARCH REPORT
## ON EUROPEAN PATENT APPLICATION NO.

EP 16 15 7534

13-07-2016

| Patent document cited in search report | | Publication date | Patent family member(s) | | Publication date |
|---|---|---|---|---|---|
| WO 2013053367 | A1 | 18-04-2013 | AU | 2011379044 A1 | 24-04-2014 |
| | | | AU | 2016202852 A1 | 26-05-2016 |
| | | | CA | 2849371 A1 | 18-04-2013 |
| | | | CN | 103997995 A | 20-08-2014 |
| | | | EP | 2765966 A1 | 20-08-2014 |
| | | | JP | 2014531294 A | 27-11-2014 |
| | | | KR | 20140078736 A | 25-06-2014 |
| | | | RU | 2014116881 A | 20-11-2015 |
| | | | US | 2014228825 A1 | 14-08-2014 |
| | | | WO | 2013053367 A1 | 18-04-2013 |
| US 2009231692 | A1 | 17-09-2009 | EP | 2093600 A1 | 26-08-2009 |
| | | | EP | 2434325 A1 | 28-03-2012 |
| | | | EP | 2434326 A1 | 28-03-2012 |
| | | | EP | 2434327 A1 | 28-03-2012 |
| | | | JP | 5287252 B2 | 11-09-2013 |
| | | | US | 2009231692 A1 | 17-09-2009 |
| | | | US | 2011141557 A1 | 16-06-2011 |
| | | | US | 2013155500 A1 | 20-06-2013 |
| | | | US | 2014211307 A1 | 31-07-2014 |
| | | | WO | 2008081729 A1 | 10-07-2008 |
| WO 2016105966 | A1 | 30-06-2016 | US | 2016175145 A1 | 23-06-2016 |
| | | | WO | 2016105966 A1 | 30-06-2016 |
| US 2007103642 | A1 | 10-05-2007 | EP | 2083672 A2 | 05-08-2009 |
| | | | EP | 2481346 A1 | 01-08-2012 |
| | | | JP | 5031035 B2 | 19-09-2012 |
| | | | JP | 2010510020 A | 02-04-2010 |
| | | | US | 2007103642 A1 | 10-05-2007 |
| | | | WO | 2008062290 A2 | 29-05-2008 |

EPO FORM P0459

**EP 3 061 430 A1**

## REFERENCES CITED IN THE DESCRIPTION

### Patent documents cited in the description

- JP 2015037415 A
- JP 2014503259 T